# EUROPEAN PATENT SPECIFICATION

(11) **EP 2 522 651 B1**
(45) Date of publication and mention of the grant of the patent: **20.11.2013**
(21) Application number: 11003905.4
(22) Date of filing: 12.05.2011
(51) Int. Cl.: C07C 37/86, C07C 39/06, C07C 37/62

(54) **Process for the purification of 2,6-diisopropyl phenol**
Verfahren zur Reinigung von 2,6-Diisopropylphenol
Procédé de purification de phénol 2,6-diisopropyle

(43) Date of publication of application: 14.11.2012
(73) Proprietor: Siegfried AG, 4800 Zofingen (CH)
(72) Inventor: Keller, Sarah, 79106 Freiburg (DE); Schlegel, Jens, 5036 Oberentfelden (CH)
(74) Representative: Isarpatent

(56) References cited:
- WO-A1-96/10004
- WO-A1-2010/067069
- DATABASE REaxys Elsevier Properties SA; XP002661042, retrieved from STN Database accession no. RX-ID: 4617515, 22108437 & NIPPON KAGAKU ZASSHI, vol. 77, 1958, page 737,

## Description

### Field of the Invention

The present invention relates to a process for a purification of 2,6-diisopropyl phenol (propofal). More particularly, the present invention relates to a purification of 2,6-diisopropyl phenol by a chlorination reaction followed by a hydrogenation reaction.

### Background of the Invention

Propofol is used as an anesthesia, which is administered Intravenously. Therefore an extremely high purification degree is necessary. Propofol Is a compound available on the market. Methods for the production of propofol are well-known In the art. Typically propofol is prepared by Friedel-Crafts alkylation from phenol and propene or 2-propanol. However, In this reaction small amounts of other Isomers and phenol derivatives than 2,6. diisopropyl phenol are formed.

The International Patent Application WO 00/34218 (Dong Kook Pharmaceutical Co., Ltd.) discloses a purification process of propofol, wherein a raw 2,6-diisopropyl phenol is treated first with an Inorganic add and second with a base, extracted with a solvent and purified by distillation or crystallization at a temperature of -20 -30°C.

EP 0 862-544 B1 (Albemarle Corporation) describes a process for the purification of propofol comprising washing the impure propofol with aqueous alkali metal hydroxide solution and/or alkali earth metal hydroxide solution in an inert atmosphere, separating the aqueous and organic phases, washing the resulting organic phase with water, and then subjecting the water-washed organic phase to distillation in an Inert environment to recover purified 2,6-diisopropyl phenol.

EP 0 716 645 B1 (Archimica S.P.A.) describes a process for purifying propofol by reacting the raw 2,6-diisopropyl phenol with an alkaline agent, Isolating the alkaline metal salt and neutralizing the isolated pure salt.

EP 0 783 473 B1 (Zambon Group S.P.A.) discloses a process for the purification of propofol comprising transformating the crude propofol into its aster with a carboxylic or sulphonic acid, crystallizing the ester and subsequent hydrolyzing it.

EP 0 511 947 B1 (Leiras Oy) discloses a process for the purification of propofol, wherein the raw 2,6-diisopropyl phenol product containing as major contaminants not more then 0.6 % by weight of 2,4-2,5-dllsopropyl-,2,4,6-triisopropyl phenol and 1-isopropoxy-2,4-diisopropyl benzene, is purified by crystallizing the same at a temperature range of -25°C to +18°C.

The International Patent Application WO 2010/067069 (University of Liverpool) discloses a method for producing halosubstituted propofol compounds (e.g. 4-bromopropofol, 4-chloropropofol). Said compounds are used as starting materials for synthesising aryl-substituted propofol analogues.

However, all the cited processes lack economical efficiency and practical usability. Moreover; the known purification processes use large amounts of solvents, Therefore there is still a need to improve the process of purifying 2,6-diisopropyl phenol.

### Summary of the Invention

The object of the present invention Is to provide an effective and ecological process for purifying 2,6-dilsopropyl phenol into a highly pure product. This object is solved by the subject-matter of the independent claims. Preferred embodiments are indicated in the dependent claims.

The purification process according to the present Invention provides 2,6-diisopropyl phenol in high yield and with a high purity In an economical effective way. One of the benefits of the present invention Is that the chlorination reaction may be carried without any solvents.

The present invention provides a process for purifying 2,6-diisopropyl phenol starting from commercially available technical grade propofol, said process comprising converting 2,6-diisopropyl phenol to a 4-chloro-2,6-diisopropyl phenol and further converting said 4-chloro-2,6-diisopropyl phenol to a purified 2,6-diisopropyl phenol.

The purification process according to the present invention is analogical applicable for purification of other 2,6-dialkyl phenols and 2,4-dialkyl phenols, In particular for purification of short-chain, preferably of C₁-C₄ 2,4-dialkyl phenols and 2,0-dialkyl phenols.

It has been surprisingly found that 2,6-diisopropyl phenol having a high degree of purity (>99.5 %) can be obtained by performing first a chlorination reaction converting the technical grade (97%) 2,6-diisopropyl phenol to a 4-chloro-2,6-diisopropyl phenol. The following hydrogenation reaction converts the 4-chloro-2,6-diisopropyl phenol into a high purity 2,6-dilsopropyl phenol.

### Detailed Description of the Invention

Hereinafter, the best mode for carrying out the present invention is described In detail.

The present invention provides an improved method for the purification of 2,6-diisopropyl phenol. Said process comprises converting technical grade (97 %) 2,6-diisopropyl phenol into 4-chloro-2,6-diisopropyl phenol and further converting said 4-chloro-2,6-diisopropyl phenol Into 2,6-diisopropyl phenol with a high degree of purity (>99.5 %:).

The process of the present invention is based on the surprising observation that a very high degree of purity (>99.5 %) of propofol can be achieved by using economical and ecological process comprising a chlorination and a hydrogenation steps, In particular, the chlorination step may be carried out without any solvents.

As known from the prior art, the purification of technical grade propofol by distillation is difficult due to the photosensitivity and oxidation sensitivity of propofol. The process according to the present invention allows the purification of 4-chloro-2,6-diisopropyl phenol by distillation, since the 4-chloropropofol Intermediate is thermal stable.

The following scheme shows the chlorination (a) and hydrogenation (b) reactions according to the present Invention:

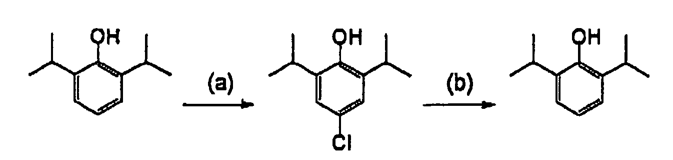

The chlorination agent may comprise any appropriate agent that permits the chlorination reaction (a). In a preferred embodiment, the chlorination agent is selected from the group of thionyl chloride, chlorine gas and sulfuryl chloride. The most preferred chlorination agent Is sulfuryl chloride.

The chlorination agent is preferably present in the reaction solution In the amount of up to 2 eq in respect to the amount of propofol, wherein eq refers to the molar equivalent. More preferably, the amount of the chlorination agent is between 1.0-1.3 eq.

In general, the chlorination reaction may be carried out In any solvent known to the man skilled In the art, preferably in a polar aprotic solvent. Most preferably, the chlorination reaction is carried out without any solvents.

The chlorination reaction is carried out at any temperature that permits the reaction to be performed, preferably at the temperature in the range of 20-100°C, more preferably in the range of 40-80°C, even more preferably in the range of 55-75°C and most preferably in the range of 60-70°C.

In a preferred embodiment, propofol and the chlorination agent are charged in a flask, optionally under Inert atmosphere, and heated for several hours. The most preferable inert gas is nitrogen. After the completion of the chlorination reaction, the resulting 4-chloro. 2,6-dllsopropyl phenol is purified according to the methods known to the man skilled in the art. Preferably the 4-chloro-2,6-dilsopropyl phenol Is purified by distillation.

The purified 4-chloro-2,6-diisopropyl phenol is further hydrogenated (b) into high purity (>99.5 %) 2,6-dllsopropyl phenol.

In a preferred embodiment, the 4-chloro-2,6-diisopropyl phenol is first dissolved in a solvent. Any appropriate solvent may be used. Preferably, the solvent is selected from the group of water, alcohols and mixtures thereof. More preferably, the solvent is selected from the group of water, methanol, ethanol, propanol, 2-propanol and mixtures thereof. Most preferably the solvent Is water.

The hydrogenation reaction (b) is preferably carried out in the presence of a base. Any inorganic or organic base may be used. Preferably, the base is selected from the group of triethylamine, diethylamine, methylamine, ethylamine, sodium hydroxide, potassium hydroxide and mixtures thereof. Most preferably the base Is sodium hydroxide.

The hydrogenation reaction (b) is preferably effected by adding a catalyst and holding the reaction mixture under hydrogen gas (H2). The hydrogen gas is applied at a pressure known to the man skilled In the art. Preferably, the pressure is up to 100 bar, more preferably in a range between 1-10 bar and most preferably in a range between 3-5 bar.

In a preferred embodiment, the catalyst is selected from a palladium or platinum catalyst. Preferably, the catalyst is a carbon supported palladium or platinum catalyst, well known in the art.

The hydrogenation reaction (b) is carried out at any temperature that permits the reaction to be performed, preferably at a temperature in a range of 0-100°C, more preferably In the range of 10-60°C and most preferably in the range of 20-30°C.

The hydrogenation reaction (b) is preferably carried out in an autoclave equipped with stirring means. The reaction mixture is hydrogenated until the reaction Is completed.

In a preferred embodiment, the catalyst is then filtered off and the organic layers are separated. The product phase (2,6-diisopropyl phenol) is preferably washed with any appropriate acid, more preferably with an Inorganic acid, optionally diluted. Most preferably, the acid is diluted hydrogen chloride. The product phase is further distilled, preferably in vacuum.

The degree of the purity of the technical grade (97 %) 2,6-diisopropyl phenol, 4-chloro-2,6-diisopropyl phenol and purified (>99.5 %).2,6-diisopropyl phenol may be measured by using GC.

The purified (>99.5 %) propofol may further be prepared into pharmaceutical dosage forms using methods known to the man skilled in the art.

Hereinafter, the present invention is illustrated In more detail by the examples, which however are not intended to limit the present Invention.

### Example 1: Purification of 2,6-diisopropyl phenol

### Step (a): Chlorination

181.7 g of sulfuryl chloride Is charged In the flask under nitrogen and heated to 70 °C. 200 g of 2,6-diisopropylphenol (technical grade) is added slowly and after complete addition the mixture Is stirred for additional 1-2 hours. The dark solution of 4-chloro-2,6-diisopropyl phenol is cooled and purified by fractionated distillation in vacuum. 167 g (70 %) of pure 4-chloro-2,6-diisopropylphenol is isolated.

### Step (b): Hydrogenation

100 g 4-chloro-2,6-diisopropylphenol is added to a mixture of 500 g of water and 70 g 30% sodium hydroxide solution at 20-25 °C. 4 g 5% palladium on charcoal (50% w/w water wet) is added and the mixture is hydrogenated at 3-5 bar hydrogen pressure for 2-4 hours at 25 °C, The catalyst is removed by filtration and the organic layers are separated. The product phase (2,6-diisopropyl phenol) Is washed with diluted hydrogen chloride once and distilled in vacuum to yield 75 g (90 %) pure 2,6-diisopropylphenol (>99.5 %).

### Example 2: Purification of 2,6-diisopropyl phenol

### Step (a): Chlorination

300 g technical grade Propofol is charged in a flask under nitrogen and heated to 70 °C. 272.5 g sulfuryl chloride is added slowly over several hours and after complete addition the mixture is stirred for additional 1-2 hours, The dark solution of 4-chloro-2,6-diisopropyl phenol is cooled and purified by fractionated distillation in vacuum. 261 g (73 %) of pure 4-chloro-2,6-diisopropylphenol is isolated.

### Step (b): Hydrogenation

100 g 4-chloro-2,6-dilsopropylphenol is added to a mixture of 500 g of water and 70 g 30% sodium hydroxide solution at 20-25 °C. 4 g 5% palladium on charcoal (60% w/w water wet) Is added and the mixture is hydrogenated at 3-5 bar hydrogen pressure for 2-4 hours at 25 °C. The catalyst Is removed by filtration and the organic layers are separated. The product phase (2,6-diisopropyl phenol) is washed with diluted hydrogen chloride once and distilled In vacuum to yield 75 g (90 %) pure 2,6-dilsopropylphenol (>99.5 %).

## Claims

1. A process for the purification of 2,6-dilsopropyl phenol, **characterized in that** the process comprises
(a) chlorinating 2,6-diisopropyl phenol to 4-chloro-2,6-diisopropyl phenol; and
(b) hydrogenating said 4-chloro-2,6-diisopropyl phenol to 2,6-disopropyl phenol

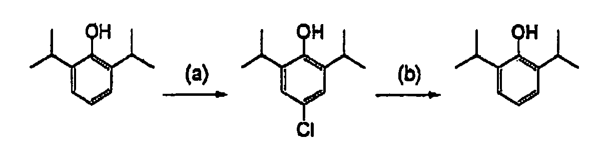

2. A process according to claim 1, **characterized in that** the chlorination reaction (a) Is carried out without any solvents.

3. A process according to any of the preceding claims, **characterized in that** the chlorination agent is selected from the group of sulfuryl chloride, thionyl chloride and chloride gas.

4. A process according to any of the preceding claims, **characterized in that** the chlorination (a) is carried out at the temperature In the range of 20-100°C, preferably in the range of 40-80°C, more preferably in the range of 55-75°C, and most preferably in the range of 60 -70°C.

5. A process according to any of the preceding claims, **characterized in that** the 4-chloro-2,6-diisopropyl phenol is further purified by distillation.

6. A process according to any of the preceding claims, **characterized in that** the hydrogenation (b) is carried out In the presence of a solvent, preferably selected from the group of water, alcohols and mixtures thereof, more preferably selected from the group of water, methanol, ethanol, propanol, 2-propanol and mixtures thereof.

7. A process according to any of the preceding claims, **characterized in that** the hydrogenation (b) is carried out in the presence of a base, preferably selected from the group of triethylamine, diethylamine, methylamine, ethylamine, sodium hydroxide, potassium hydroxide and mixtures thereof.

8. A process according to any of the preceding claims, **characterized in that** the hydrogenation (b) is carried out in the presence of a catalyst, preferably the catalyst is selected from a palladium or platinum catalyst.

9. A process according to any of the preceding claims, **characterized in that** the hydrogenation (b) is carried out at a temperature in the range of 0-100°C, preferably in the range of 10-60°C and most preferably in the range of 20-30°C.

10. A process according to any of the preceding claims, **Characterized in that** the hydrogenated 2,6-diisopropyl phenol is further purified by vacuum distillation.

## Patentansprüche

1. Verfahren zum Reinigen von 2,6-Diisopropylphenol, **dadurch gekennzeichnet, dass** das Verfahren Folgendes umfasst:
(a) Chlorieren von 2,6-Diisopropylphenol zu 4-Chlor-2,6-diisopropylphenol; und
(b) Hydrieren des 4-Chlor-2,6-diisopropylphenols zu 2,6-Diisopropylphenol:

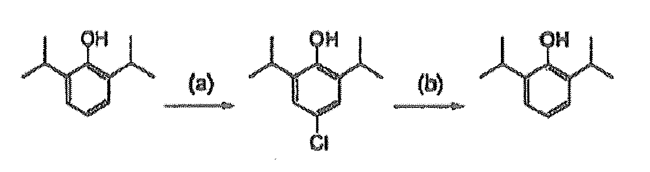

2. Verfahren nach Anspruch 1, **dadurch gekennzeichnet, dass** die Chlorierungsreaktion (a) ohne Lösemittel ausgeführt wird.

3. Verfahren nach einem der vorangehenden Ansprüche, **dadurch gekennzeichnet, dass** das Chlorierungsmittel aus der Gruppe Sulfurylchlorid, Thionylchlorid und Chloridgas ausgewählt wird.

4. Verfahren nach einem der vorangehenden Ansprüche, **dadurch gekennzeichnet, dass** die Chlorierung (a) bei einer Temperatur im Bereich von 20-100 °C, bevorzugt im Bereich von 40-80 °C, besonders bevorzugt im Bereich von 55-75 °C und ganz besonders bevorzugt im Bereich von 60-70 °C ausgeführt wird.

5. Verfahren nach einem der vorangehenden Ansprüche, **dadurch gekennzeichnet, dass** das 4-Chlor-2,6-diisopropylphenol durch Destillation weiter gereinigt wird.

6. Verfahren nach einem der vorangehenden Ansprüche, **dadurch gekennzeichnet, dass** die Hydrierung (b) in Gegenwart eines Lösemittels ausgeführt wird, das bevorzugt aus der Gruppe Wasser, Alkohole und Gemischen davon ausgewählt wird und besonders bevorzugt aus der Gruppe Wasser, Mealsol, Ealsol, Propanol, 2-Propanol und Gemischen davon ausgewählt wird.

7. Verfahren nach einem der vorangehenden Ansprüche, **dadurch gekennzeichnet, dass** die Hydrierung (b) in Gegenwart einer Base ausgeführt wird, die bevorzugt aus der Gruppe Triethylainin, Diethylamin, Methylamin, Ethylemin, Natriumhydroxid, Kaliumhydroxid und Gemischen davon ausgewählt wird.

8. Verfahren nach einem der vorangehenden Ansprüche, **dadurch gekennzeichnet, dass** die Hydrierung (b) in Gegenwart eines Katalysators ausgeführt wird, wobei der Katalysator bevorzugt aus einem Palladium- oder Platin-Katalysator ausgewählt wird.

9. Verfahren nach einem der vorangehenden Ansprüche, **dadurch gekennzeichnet, dass** die Hydrierung (b) bei einer Temperatur im Bereich von 0-100 °C, bevorzugt im Bereich von 10-60 °C und besonders bevorzugt im Bereich von 20-30 °C ausgeführt wird.

10. Verfahren nach einem der vorangehenden Ansprüche, **dadurch gekennzeichnet, dass** das hydrierte 2,6-Diisopropylphenol durch Vakuumdestillation weiter gereinigt wird.

## Revendications

1. Procédé pour la purification du phénol de 2,6-diisopropyle, **caractérisé en ce que** le procédé comprend :
(a) la chloration du phénol de 2,6-diisopropyle en phénol de 4-chloro-2,6-dipropyle ; et
(b) l'hydrogénation dudit phénol de 4-chloro-2,6-diisopropyle en phénol de 2,6-diisopropyle

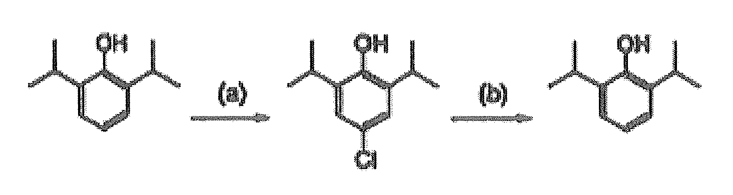

2. Procédé selon la revendication 1, **caractérisée en ce que** la réaction de chloration (a) est réalisée sans aucun solvant.

3. Procédé selon l'une quelconque des revendications précédentes, **caractérisé en ce que** l'agent de chloration est choisi parmi le groupe du chlorure de sulfuryle, du chlorure de thionyle et du chlorure gazeux.

4. Procédé selon l'une quelconque des revendications précédentes, **caractérisé en ce que** la chloration (a) est réalisée à la température située dans la plage allant de 20 à 100°C, de préférence dans la plage allant de 40 à 80°C, davantage de préférence dans la plage de 55 à 75°C et de préférence entre toutes dans la plage de 60 à 70°C.

5. Procédé selon l'une quelconque des revendications précédentes, **caractérisé en ce que** le phénol de 4-chloro-2,6-diisopropyle est encore purifié par distillation.

6. Procédé selon l'une quelconque des revendications précédentes, **caractérisé en ce que** l'hydrogénation (b) est réalisée en présence d'un solvant, de préférence choisi parmi le groupe de l'eau, des alcools et mélanges associés, de préférence choisi parmi le groupe de l'eau, du méthanol, de l'éthanol, du propanol, du 2-propanol et des mélanges associés.

7. Procédé selon l'une quelconque des revendications précédentes, **caractérisé en ce que** l'hydrogénation (b) est réalisée en présence d'une base, de préférence choisie parmi le groupe de la triéthylamine, de la diéthylamine, de la méthylamine, de l'éthylamine, de l'hydroxyde de sodium, de l'hydroxyde de potassium et des mélanges associés.

8. Procédé selon l'une quelconque des revendications précédentes, **caractérisé en ce que** l'hydrogénation (b) est réalisée en présence d'un catalyseur, de préférence le catalyseur est choisi parmi un catalyseur de palladium ou de platine.

9. Procédé selon l'une quelconque des revendications précédentes, **caractérisé en ce que** l'hydrogénation (b) est réalisée à une température dans la plage allant de 0 à 100°C, de préférence dans la plage allant de 10 à 60°C et de préférence entre toutes dans la plage de 20 à 30°C.

10. Procédé selon l'une quelconque des revendications précédentes, **caractérisé en ce que** le phénol de 2,6-diisopropyle est encore purifié par distillation sous vide.
